# NEUE EUROPÄISCHE PATENTSCHRIFT

(11) **EP 0 567 448 B2**
(45) Veröffentlichungstag und Bekanntmachung des Hinweises auf die Entscheidung über den Einspruch: **05.01.2005**
(45) Hinweis auf die Patenterteilung: 03.06.1998
(21) Anmeldenummer: 93890076.8
(22) Anmeldetag: 07.04.1993
(51) Int. Cl.: C07K 14/435, A61K 38/37

(54) **Verfahren zur Herstellung von Faktor VIII**
Factor VIII preparation
Préparation de facteur VIII

(30) Priorität: 24.04.1992 AT 84992
(43) Veröffentlichungstag der Anmeldung: 27.10.1993
(73) Patentinhaber: IMMUNO Aktiengesellschaft, A-1221 Wien (AT)
(72) Erfinder: Eibl, Johann, Dr., A-1180 Wien (AT); Elsinger, Friedrich, Dr., A-1130 Wien (AT); Gritsch, Herbert, A-1140 Wien (AT); Linnau, Yendra, Dr., A-1224 Wien (AT); Schwarz, Otto, Dr., A-1190 Wien (AT); Turecek, Peter, Dr., A-1190 Wien (AT); Wöber, Günther, Prof. Dr., A-2522 Oberwaltersdorf (AT)
(74) Vertreter: Sonn, Helmut

(56) Entgegenhaltungen:
- EP-A- 0 131 740
- EP-A- 0 144 709
- EP-A- 0 378 208
- EP-A- 0 519 901
- EP-A- 0 534 812
- Blum and Thomas, Haemostasis and Thrombosis,Churchill Livingstone, New York, 1981 pp, 836-937
- Mannucci, The Lancet, vol. 339, p. 819, 1992,

## Beschreibung

Die Erfindung betrifft ein Verfahren zur Herstellung eines virussicheren, hochgereinigten Faktor VIII .

Die Blutgerinnung wird durch eine Reihe von aufeinanderfolgenden Reaktionen verschiedener Proteine bzw. Enzyme ausgelöst. Durch einen Mangel an Blutgerinnungsfaktoren wird die Bildung von Fibrin aus Fibrinogen und damit der Wundverschluß verhindert; die Folge sind Blutungen. Ein solcher Fall liegt bei der Hämophilie A vor. Diese ist die am meisten verbreitete Blutungskrankheit und wird durch den Mangel an Faktor VIII hervorgerufen. Der Faktor VIII kommt im Plasma gemeinsam mit dem von Willebrand Faktor (vWF) als nicht kovalent gebundener Komplex (FVIII/vWF) vor. Die Proteine FVIII und vWF, sowie der Komplex FVIII/vWF werden zur Substitutionsbehandlung Hämophiler verwendet. An die entsprechenden pharmazeutischen Präparate werden hohe Anforderungen hinsichtlich Wirksamkeit und Sicherheit gestellt.

Ausgangsmaterial zur Herstellung der Präparate ist zumeist menschliches Plasma, das den Faktor VIII aber nur in geringen Mengen enthält (etwa 0,1 - 0,2 µg/ml). Zur Gewinnung des Faktor VIII müssen daher nicht nur große Mengen Plasma verarbeitet werden, es müssen auch die störenden Begleitproteine so weit wie möglich abgetrennt werden, wobei viele dieser Begleitproteine ähnliche physikalisch-chemische Eigenschaften aufweisen.

Eine weitere Schwierigkeit besteht darin, daß das Präparat noch hinsichtlich infektiöser Agentien inaktiviert werden muß, da das Ausgangsmaterial z.B. Hepatitis-Virus oder HIV enthalten kann. Dazu kommt noch, daß jeder einzelne Verfahrensschritt und jede Inaktivierung so durchgeführt werden müssen, daß die biologische Aktivität der gewünschten Gerinnungsfaktoren möglichst erhalten bleibt.

Bei den klassischen Produktionsmethoden kommen stufenweise Präzipitationen (z.B. Kryopräzipitation oder Fällungen durch Zusatz von Ammoniumsulfat) zum Einsatz, deren Zweck Entfernung der Verunreinigungen, wie Prothrombinkomplexfaktoren, Fibrinogen und Fibronectin ist. Die Reinheit der erhaltenen Faktor VIII-Konzentrate liegt etwa bei 1 E/mg Protein und übersteigt üblicherweise die Grenzen von 10-20 E/mg Protein nicht.

Aus der EP-A-0 378 208 ist ein Verfahren zur Herstellung von Faktor VIII bekannt, nach dem eine Kryopräzipitat-Lösung einer Behandlung mit einem organischen Lösungsmittel (Tri-(n-butyl)phosphat (TNBP)) in Gegenwart des Lösungsvermittlers Tween®80 zur Virusinaktivierung unterworfen wird. Der Faktor VIII wird anschließend einer zweistufigen Reinigungsfällung unterworfen, lyophilisiert und im trockenen Zustand erhitzt.

Die Behandlung biologischer und pharmazeutischer Produkte mit 0,25 - 10 Gew.% eines nicht denaturierenden Amphiphils (Detergens) ist in der EP-B-0 050 061 beschrieben.

Das Verfahren zur Behandlung von Proteinen mit organischen Lösungsmitteln, gegebenenfalls in Gegenwart von Detergenzien, ist durch die EP-B-0 131 740 bekannt. Hier wird gezeigt, daß die Behandlung mit einem Detergens alleine bezüglich der Virusinaktivierung relativ unwirksam ist. Organische Lösungsmittel wirken jedoch nur gegen membranumhüllte Viren. Es sind bereits Hepatitis-Infektionen aufgetreten, die auf mit Hepatitis A kontaminierte Faktor VIII-Konzentrate zurückgeführt werden, welche auf diese Weise virusinaktiviert worden sind. Hepatitis A-Virus besitzt keine Lipid-hältige Membran; es wird daher nicht inaktiviert (Mannucci P M et al (1992) The Lancet 339, 819 "Outbreak of hepatitis A among Italian patients with haemophilia").

Die Behandlung von Faktor VIII-hältigen Fraktionen mit organischen Lösungsmitteln wird meist derart durchgeführt, daß das toxisch wirkende Lösungsmittel nach erfolgter Behandlung in aufwendiger Weise abgetrennt werden muß. In der EP-A-0 343 275 wird zu diesem Zweck die Extraktion der Faktor VIII-hältigen Fraktion mit Ölen, wie Sojaöl oder Rizinöl, vorgeschlagen. Danach wird Faktor VIII einer Gelpermeationschromatographie an Ionenaustauschermaterialien unterworfen.

Nach dem Verfahren der EP-A-0 094 611 wird Faktor VIII zur Reduktion der Infektiosität im trockenen Zustand erhitzt.

Gemäß der EP-B-0 159 311 werden Blutprodukte im festen, feuchten Zustand zur Inaktivierung potentiell vorhandener Viren erhitzt. Für ein derart "dampferhitztes" Faktor VIII-Konzentrat ist kein Fall einer Infektion bekannt (Mannucci P M (1992) Transfusion 32, 134-138 "Low risk of viral infection after administration of vaporheated factor VIII concentrates").

Eine Hitzebehandlung hochgereinigter Faktor VIII-Konzentrate gilt als kritisch. Einem chromatographisch gereinigten Faktor VIII muß gemäß der EP-A-0 173 242 beispielsweise ein Gemisch von Stabilisatoren (Kohlenhydrate und Aminosäuren) zugesetzt werden, bevor dieser in Lösung erhitzt wird. Ansonsten ist es üblich, die Faktor VIII-hältige Fraktion noch vor der chromatographischen Reinigung einer Behandlung zur Virusinaktivierung zu unterziehen.

Die Erfindung stellt sich zur Aufgabe, ein Verfahren zur Verfügung zu stellen, nach dem ein gereinigter Faktor VIII, der auf Grund einer effektiven Behandlung zur Virus Inaktivierung als virussicher gilt, hergestellt werden kann.

Vorzugsweise soll die spezifische Aktivität des hergestellten Präparates mindestens 25 E/mg Protein betragen.

Die Aufgabe wird erfindungsgemäß durch die Kombination der folgenden Maßnahmen gelöst:
a) einer chromatographischen Reinigung einer Faktor VIII-hältigen Fraktion,
b) einer Tensidbehandlung des Faktors VIII in wässeriger Lösung, welche frei von organischen Lösungsmitteln ist, bei einem Tensid/Protein-Verhältnis von 1:1 bis 1000:1 und
c) einer Hitzebehandlung der Faktor VIII-Präparation in festem Zustand.

Faktor VIII kann erfindungsgemäß als FVIII/vWF-Komplex, FVIII bzw. vWF hergestellt werden. Während des Herstellungsverfahrens kann etwa eine Behandlung zur Dissoziation des FVIII/vWF - Komplexes vorgenommen werden, z.B. mit Calciumchlorid.

Es wurde gefunden, daß die erfindungsgemäße Tensidbehandlung überraschenderweise zur erfolgreichen Virusinaktivierung herangezogen werden kann. Als Tenside werden beispielsweise nichtionische Tenside, wie Tween® 80, Triton® X-100, Triton® X-114, Dodecylmaltosid oder Octylglucosid, zwitterionische Tenside, wie Dimethyloctylamin-N-oxid oder N-Dodecyl-N,N-dimethyl-3-ammonio-1-propansulfonat, und ionische Tenside, wie Natriumdesoxycholat oder Natriumcholat. eingesetzt. Die Tensidbehandlung wird vor allem in wässeriger Lösung, welche frei von organischen Lösungsmitteln ist, vorgenommen. Als wässerige Lösung bietet sich etwa eine Kryopräzipitatlösung an, die gegebenenfalls noch durch Adsorption der Prothrombinkomplex-Faktoren gereinigt ist. In einer solchen Lösung wird das Tensid/Protein-Verhältnis von 1:1 bis 10:1 bevorzugt.

Die Tensidbehandlung wird vorzugsweise vor der chromatographischen Reinigung, vorzugsweise mit einem Tensid/Protein-Verhältnis von 3,5:1 bis 10:1, durchgeführt, wobei das Tensid während der Chromatographie in einfacher Weise abgetrennt werden kann.

In einer weiteren Ausführungsform ist aber auch die Tensidbehandlung während der Chromatographie möglich. Diese Variante hat den Vorteil, daß Faktor VIII auch in Gegenwart von zur Aggregation und somit zur unerwünschten Coadsorption neigenden Proteinen in spezifischer Weise adsorbiert wird.

Wenn von Faktor VIII-hältigem Ausgangsmaterial mit geringem Proteingehalt ausgegangen wird, ist es oft angebracht, eine bestimmte Tensidkonzentration in der wässerigen Lösung aufrecht zu erhalten. Dadurch kann das Tensid/Protein-Verhältnis bis zu 1000:1 ausmachen.

Ein Zellkulturüberstand, der rekombinanten Faktor VIII enthält, weist beispielsweise einen Proteingehalt von mindestens etwa 100 mg/l auf. Eine zehnprozentige Tensidkonzentration in diesem Kulturüberstand entspricht daher einem Tensid/Protein-Verhältnis von nicht mehr als etwa 1000:1.

Um die Sicherheit gegenüber einer Infektion durch nicht-membranumhüllte Viren zu erhöhen, wird die erfindungsgemäße Kombination mit der Hitzebehandlung des Faktor VIII im festen Zustand vorgeschlagen. Diese kann am lyophilisierten Präparat vorgenommen werden, wobei die Hitzebehandlung vorzugsweise im feuchten Zustand des Präparats erfolgt.

Eine bevorzugte Ausführungsform der Erfindung schließt daher die Hitzebehandlung mit heißem Dampf ein, wobei die Faktor VIII-Präparation im festen Zustand auf einen Gehalt an Wasser, Methanol oder Ethanol von mehr als 0,05 (5 % Masse) und weniger als 0,70 (70 % Masse), vorzugsweise weniger als 0,40 (40 % Masse), eingestellt und in einem geschlossenen Behälter, gegebenenfalls in Gegenwart eines inerten Schutzgases, bei einer Temperatur im Bereich von 50 bis 121°C erhitzt wird.

Im Faktor VIII-Lyophilisat können weiters Albumin bzw. Salze, wie Natriumcitrat, bzw. Aminosäuren enthalten sein.

Es hat sich überraschenderweise herausgestellt, daß diese Hitzebehandlung keine wesentlichen Aktivitätseinbußen mit sich bringt, weshalb auch ein relativ instabiler, hochgereinigter Faktor VIII behandelt werden kann.

Während der chromatographischen Reinigung wird Faktor VIII von den Begleitproteinen weitgehend befreit. Eine bevorzugte Ausführungsform des Herstellungsverfahrens umfaßt eine mehrstufige chromatographische Reinigung, wobei in einer Stufe ein Adsorptionsmaterial für Faktor VIII eingesetzt wird und in einer weiteren Stufe ein Material zur Adsorption von verunreinigenden Proteinen verwendet wird.

So kann etwa zuerst ein Anionenaustauscher zur Adsorption von Faktor VIII und anschließend ein Material mit hoher Affinität für Fibronectin, wie Gelatine oder Heparin, immobilisiert an einem unlöslichen Träger, eingesetzt werden. Auch hier kann die Chromatographie in Gegenwart von Tensiden vorteilhaft sein, um die unspezifische Adsorption von Faktor VIII an den Affinitätsträger zu minimieren.

Für die Herstellung einer albuminfreien Faktor VIII-Präparation kann die Adsorption der Faktor VIII-hältigen Lösung an Triazingele (Cibacron®-Blue 3GA, immobilisiert an einem unlöslichen Träger (Fa. Bio-Rad); Fractogel® TSK-AF-Blue (Fa. Merck), Blue-Sepharose®-CL6B (Fa. Pharmacia)) erfolgen.

Derartige Gele binden mit hoher Selektivität Albumin, welches quantitativ mit anderen Methoden nicht vom Faktor VIII zu trennen ist und als Verunreinigung in Faktor VIII-Präparationen vorliegt, sofern ein Albumingehalt zur Stabilisierung nicht erwünscht ist.

Bevorzugt werden für die chromatographische Reinigung Anionenaustauscher auf Acrylat-, Silikat- oder Kohlenhydratbasis, etwa DEAE-Sephadex® (Fa. Pharmacia), QAE-Sepharose® (Fa. Pharmacia), DEAE-Toyo-Pearl® (Fa. Tosohaas), TMAE-Fractogel® (Fa. Merck) und dergleichen, verwendet.

Die Anionenaustauscher auf Acrylatbasis sind vorzugsweise als "Tentakelmatrix" ausgebildet (vergleiche Müller W (1990) Journal of Chromatography 510, 133-140 "New ion exchangers for the chromatography of biopolymers"). Bei einer solchen Matrix befinden sich die Austauschergruppen entlang einer polymeren Seitenkette, die mit der Matrixoberfläche verbunden ist. Daraus resultiert eine hohe Beladungskapazität und eine verbesserte Selektivität.

Der Blutgerinnungsfaktor VIII/vwF-komplex, welcher nach dem erfindungsgemäßen Verfahren hergestellt ist, gilt als virussicher und hat vorzugsweise eine hohe spezifische Aktivität von mindestens 25 E/mg Protein.

Die Erfindung wird durch die nachfolgenden Beispiele noch näher erläutert.

### Beispiel 1

1 kg Kryopräzipitat, gewonnen aus 101 Liter Plasma, wurde in 3,5 Liter Puffer 1, eine Tris-Lysin gepufferte NaCl-Lösung (85 mmol/l), gelöst.

Zur Vorreinigung der Faktor VIII-hältigen Lösung wurde Al(OH)₃ eingerührt, das Al(OH)₃ abgetrennt, BaSO₄ eingerührt und das BaSO₄ abgetrennt. Anschließend wurde der pH-Wert des Überstandes auf 6,5 eingestellt, die Temperatur auf 4°C gesenkt und der entstandene Niederschlag durch Zentrifugieren abgetrennt und verworfen.
110 g Triton®X-100 pro Liter Überstand (Tensid/Protein-Verhältnis von 55:1) wurde zugesetzt und 30 min bei 25°C gerührt. Faktor VIII wurde dann bei pH 6,8 an 750 ml DEAE-Toyopearl® 650 M der Fa. Tosohaas, äquilibriert mit Puffer 1, in einer Säule mit einem Durchmesser von 9 cm adsorbiert.

Die Faktor VIII-hältige Fraktion wurde mit 2,25 Liter Puffer 2 (eine Citrat-gepufferte 500 mM NaCl-Lösung) vom Gel eluiert.

Die spezifische Aktivität betrug 76 IE Faktor VIII pro mg Protein.

Nach der Gefriertrocknung der ultrafiltrierten Faktor VIII-hältigen Fraktion wurde der Faktor VIII mit 7.5 % w/w H₂O in einem geschlossenen Behälter 10 Stunden bei 60°C erhitzt. Die Faktor VIII-Aktivität betrug 72 % der nicht erhitzten Probe. Eine Hitzebehandlung in Anwesenheit von Albumin führte zu einer Faktor VIII-Aktivität von 92 % der nicht erhitzten Probe.

### Beispiel 2

1 kg Kryopräzipitat wurde in der gleichen Weise wie in Beispiel 1 gewonnen und gelöst.

Die Vorreinigung wurde mit Al(OH)₃ und BaSO₄ durchgeführt. Zum Überstand wurden 120 g Tween 80 pro Liter zugesetzt (Tensid/Protein-Verhältnis von 4:1) und 45 min bei 25°C gerührt. Nach dem Verdünnen mit Puffer 1 im Verhältnis 1:3 wurde die Lösung durch Adsorption des Faktor VIII an 400 ml EMD-TMAE-Fractogel® (Fa. Merck) chromatographisch gereinigt. Das Gel wurde vorher mit Puffer 1 äquilibriert. Die nicht gebundenen Proteine wurden mit Puffer 1 abgetrennt, anschließend wurde die Faktor VIII-hältige Fraktion mit Puffer 2 eluiert.

Die spezifische Aktivität betrug 45 IE Faktor VIII pro mg Protein.

Das Eluat wurde durch Ultrafiltration aufkonzentriert und gefriergetrocknet. In einem geschlossenen Behälter wurde der Faktor VIII in Anwesenheit von 9,5 % w/w H₂O 10 Stunden bei 60°C erhitzt.

Die Faktor VIII-Aktivität betrug 87 % der nicht erhitzten Probe.

Der Gesamt-Virus-Reduktionsfaktor gemäß der Richtlinie EC III/8115/89-EN der Kommission der Europäischen Gemeinschaften wurde am Beispiel von HIV-1 und FSME-Viren bestimmt, wobei Virussuspension während dieses Verfahrens mehrfach zugesetzt worden ist. Der Gesamt-Virus-Reduktionsfaktor betrug mindestens 12. Dies entspricht einer Reduktion des theoretischen Virustiters von mindestens 10¹² über das gesamte Verfahren.

### Beispiel 3

Die Präparation mit 3000 IE Faktor VIII, gemäß Beispiel 2 hergestellt, enthielt 120 E Fibronectin (3960 µg).

Zur Entfernung dieser Verunreinigung wurde die Faktor VIII-hältige Präparation mit einer 15 ml Heparin-Sepharose Säule (1,6 cm Durchmesser) mit einem Puffer von 260 mM NaCl chromatographisch gereinigt.

Faktor VIII wurde nicht gebunden, Fibronectin war in der Faktor VIII-hältigen Fraktion nicht nachweisbar. Die spezifische Aktivität betrug 46 IE Faktor VIII pro mg Protein.
Fibronectin konnte durch Eluieren mit einer 750 mM NaCl-Lösung gewonnen werden.

### Beispiel 4

1 kg Kryopräzipitat wurde in der gleichen Weise wie in Beispiel 1 gewonnen und gelöst.
Die Vorreinigung wurde mit Al(OH)₃ und PEG 4000 durchgeführt.

0,15 % Al(OH)₃ wurde zugesetzt und 30 min bei 25°C gerührt. Al(OH)₃ wurde dann durch Zentrifugierten abgetrennt und verworfen.
Zum Überstand wurden 3.15 % PEG 4000 zugesetzt, der pH-Wert auf 6,6 gestellt und die Temperatur auf 9°C gesenkt. 30 min wurde gerührt und der entstandene Niederschlag durch Zentrifugieren abgetrennt und verworfen.

Zum Überstand wurden weiters 100 g Tween 80 pro Liter zugesetzt (Tensid/Protein-Verhältnis von 50:1) und gut durchgemischt. Nach Verdünnung mit Puffer 1 im Verhältnis 1 zu 3 wurde die Lösung an 1,75 Liter Q-Sepharose® Fast Flow der Fa. Pharmacia chromatographiert.

Faktor VIII wurde adsorbiert und die ungebundenen Proteine mit 7,9 Liter Puffer 1 abgetrennt. Durch Eluieren mit 5.25 Liter einer Natriumcitrat-gepufferten 500 mM NaCl-Lösung wurde eine Faktor VIII-hältige Fraktion erhalten.

Die spezifische Aktivität betrug 89 IE Faktor VIII pro mg Protein.

Das ankonzentierte Eluat wurde lyophilisiert und anschließend in Anwesenheit von 8 % w/w H₂O in einem geschlossenen Gefäß 10 Stunden bei 60°C erhitzt.

Die Faktor VIII-Aktivität betrug 81 % der nicht erhitzten Probe.

## Patentansprüche

1. Verfahren zur Herstellung einer hochgereinigten, virussicheren Faktor VIII-Präparation, **gekennzeichnet durch** die Kombination der Maßnahmen
a) einer chromatographischen Reinigung einer Faktor VIII-hältigen Fraktion,
b) einer Tensidbehandlung des Faktors VIII in wässeriger Lösung, welche frei von organischen Lösungsmitteln ist, bei einem Tensid/Protein-Verhältnis von 1:1 bis 1000:1 und
c) einer Hitzebehandlung der Faktor VIII-Präparation in festem Zustand.

2. Verfahren nach Anspruch 1, **dadurch gekennzeichnet, daß** die Tensidbehandlung vor der chromatographischen Reinigung vorgenommen wird, wobei das Tensid/Protein-Verhältnis 3,5:1 bis 10:1 beträgt.

3. Verfahren nach Anspruch 1, **dadurch gekennzeichnet, daß** die Tensidbehandlung während der chromatographischen Reinigung vorgenommen wird.

4. Verfahren nach einem der Ansprüche 1 bis 3, **dadurch gekennzeichnet, daß** die Hitzebehandlung mit heißem Dampf erfolgt, wobei die Faktor VIII-Präparation in festem Zustand auf einen Gehalt an Wasser, Methanol oder Ethanol von mehr als 0,05 (5 % Masse) und weniger als 0,70 (70 % Masse), vorzugsweise weniger als 0,40 (40 % Masse), eingestellt und in einem geschlossenen Behälter, gegebenenfalls in Gegenwart eines inerten Schutzgases, bei einer Temperatur im Bereich von 50 bis 121°C behandelt wird.

5. Verfahren nach einem der Ansprüche 1 bis 4, **dadurch gekennzeichnet, daß** die chromatographische Reinigung mehrstufig vorgenommen wird, wobei in einer Stufe ein Material eingesetzt wird, welches Faktor VIII adsorbiert, und in einer weiteren Stufe ein Material zur Adsorption von verunreinigenden Proteinen verwendet wird.

6. Verfahren nach einem der Ansprüche 1 bis 6, **dadurch gekennzeichnet, daß** für die chromatographische Reinigung ein Anionenaustauscher auf Acrylat-, Silikat- oder Kohlenhydratbasis eingesetzt wird.

7. Verfahren nach Anspruch 6, **dadurch gekennzeichnet, daß** ein Anionenaustauscher eingesetzt wird, welcher aus einer Matrix besteht, an die polymere Seitenketten, die Anionenaustauschergruppen aufweisen, gebunden sind.

8. Blutgerinnungsfaktor VIII/vWF - KOMPLEX-Präparation, hergestellt nach einem der Ansprüche 1 bis 7, mit einer spezifischen Aktivität von mindestens 25 E/mg Protein.

## Claims

1. A method of producing a highly purified virus-safe factor VIII preparation, **characterised by** the combination of the measures
a) chromatographic purification of a factor VIII-containing fraction,
b) tenside treatment of factor VIII in an aqueous solution which is free from organic solvents and at a tenside/protein ratio of from 1:1 to 1000:1, and
c) heat treatment of the factor VIII preparation in the solid state.

2. A method according to claim 1, **characterised in that** the tenside treatment is carried out prior to chromatographic purification, wherein the tenside/protein ratio is from 3.5:1 to 10:1.

3. A method according to claim 1, **characterised in that** the tenside treatment is carried out during the chromatographic purification.

4. A method according to any one of claims 1 to 3, **characterised in that** the heat treatment is effected with hot vapour, wherein the factor VIII preparation in the solid state is adjusted to a content of water, methanol or ethanol of above 0.05 (5 % by mass) and below 0.70 (70 % by mass), preferably below 0.40 (40 % by mass), and is treated in a closed container, optionally in the presence of an inert protective gas, at a temperature ranging between 50 and 121°C.

5. A method according to any one of claims 1 to 4, **characterised in that** the chromatographic purification is carried out in several steps, wherein a material is employed in one step which adsorbs factor VIII, and a material for the adsorption of contaminating proteins is used in a further step.

6. A method according to any one of claims 1 to 6, **characterised in that** for chromatographic purification, an anion exchanger based on acrylates, silicates or carbohydrates is used.

7. A method according to claim 6, **characterised in that** an anion exchanger is used which is comprised of a matrix to which polymer side chains are bonded, the polymer side chains comprising anion exchanger groups.

8. Blood coagulation factor VIII/vWF-Komplex preparation prepared according to any one of claims 1 to 7, having specific activity of at least 25 U/mg protein.

## Revendications

1. Procédé de production d'une préparation de facteur VIII très purifiée, exempte de virus, **caractérisé par** la combinaison des mesures
a) purification par chromatographie d'une fraction contenant le facteur VIII,
b) traitement par tensioactif du facteur VIII en solution aqueuse exempte de solvants organiques, pour un rapport tensioactif/protéine de 1 : 1 à 1000 : 1 et
c) traitement thermique de la préparation de facteur VIII à l'état solide.

2. Procédé selon la revendication 1, **caractérisé en ce que** le traitement par tensioactif est mis en oeuvre avant la purification par chromatographie, le rapport tensioactif/protéine étant de 3,5 : 1 à 10 : :1.

3. Procédé selon la revendication 1, **caractérisé en ce que** le traitement par tensioactif est mis en oeuvre pendant la purification par chromatographie.

4. Procédé selon une des revendications 1 à 3, **caractérisé en ce que** le traitement thermique s'effectue au moyen de vapeur chaude, la préparation de facteur VIII à l'état solide étant ajustée à une teneur en eau, méthanol ou éthanol de plus de 0,05 (5 % en masse) et moins de 0,70 (70 % en masse), de préférence moins de 0,40 (40 % en masse), et étant traitée dans un récipient fermé, le cas échéant en présence d'un gaz protecteur inerte, à une température de l'ordre de 50 à 121°C.

5. Procédé selon une des revendications 1 à 4, **caractérisé en ce que** la purification par chromatographie s'effectue en plusieurs étapes, une substance adsorbant le facteur VIII étant utilisée dans une étape et une substance adsorbant les protéines contaminantes étant utilisée dans une autre étape.

6. Procédé selon une des revendications 1 à 6, **caractérisé en ce qu'**on utilise pour la purification par chromatographie un échangeur d'anions à base d'acrylate, de silicate ou de glucide.

7. Procédé selon la revendication 6, **caractérisé en ce qu'**on utilise un échangeur d'anions composé d'une matrice à laquelle sont liées des chaînes latérales polymères présentant des groupes échangeurs d'anions.

8. Préparation de facteur VIII/vWF-complexe de la coagulation sanguine, produite selon une des revendications 1 à 7, ayant une activité spécifique d'au moins 25 U/mg de protéine.
